# EUROPEAN PATENT SPECIFICATION

(11) **EP 2 428 202 B1**
(45) Date of publication and mention of the grant of the patent: **01.07.2015**
(21) Application number: 11181153.5
(22) Date of filing: 13.09.2011
(51) Int. Cl.: A61K 9/00, A61K 31/4184, A61P 9/12

(54) **Orally disintegrating compositions**
Oral lösliche Zusammensetzungen
Compositions à désintégration orale

(30) Priority: 14.09.2010 TR 201007508
(43) Date of publication of application: 14.03.2012
(73) Proprietor: Sanovel Ilaç Sanayi Ve Ticaret Anonim Sirketi, 34460 Sariyer/Istanbul (TR)
(72) Inventor: Cifter, Ümit, 34398 Istanbul (TR); Türkyilmaz, Ali, 34398 Istanbul (TR); Mutlu, Onur, 34398 Istanbul (TR); Ramazanoglu, Gaye, 34398 Istanbul (TR)
(74) Representative: Sevinç, Erkan

(56) References cited:
- EP-A1- 2 394 638
- WO-A1-98/53798
- WO-A1-99/25321
- WO-A2-2010/075347
- DE-A1-102006 027 794
- GB-A- 2 460 915

## Description

### Technical Aspect

The present invention is related to an orally disintegrating composition comprising compound of Formula I (Compound I) or pharmaceutically acceptable salts and/or esters thereof and one or more pharmaceutically acceptable excipient.

Furthermore, the invention relates to methods for preparing the orally disintegrating compositions comprising Compound I and its use for preventing and/or treating hypertension in mammals, particularly in humans.

### Background of the Invention

Hypertension affects about 20% of the adult population in developed countries. In the adult population aged 60 years or older, this percentage increases to about 60% to 70% in general. Hypertension also is associated with an increased risk of other physiological complications including stroke, myocardial infarction, atrial fibrillation, heart failure, peripheral vascular disease and renal impairment. Although a number of antihypertensive drugs are available in various pharmacological categories, the efficacy and safety of such drugs can vary from patient to patient, in this regard new treatments are still a desired subject.

Azilsartan which has a chemical name as 2-ethoxy-1-((2'-(5-oxo-2,5-dihydro-1,2,4-oxadiazol-3yl)-biphenyl-4-yl)methyl)-1 H-benzimidazole-7-carboxylic acid (hereinafter referred as "Compound I") is an angiotensin II receptor antagonist and its chemical structure is shown in the Formula I.

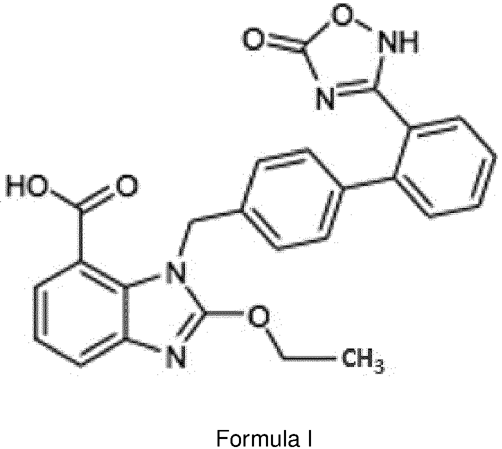

Angiotensin II receptor antagonists are used in the management of hypertension; they may have a particular role in patients who develop cough with ACE inhibitors. Some are also used in diabetic nephropathy and in the management of heart failure. They act mainly by selective blockade of AT1 receptors thus reducing the pressor effects of angiotension II. Known angiotension receptor II antagonists from the prior art are candesartan, eprosartan, irbesartan, losartan, olmesartan, telmisartan and valsartan.

In prior art, there are also several patents which disclose orally disintegrating compositions but none of them includes Compound I or pharmaceutically acceptable salts and/or esters thereof.

Various formulations and methods are already known for the preparation of orally disintegrating formulations. However, orally disintegrating formulations are becoming an increasingly important issue in the area of better patient compliance comparative to the conventional solid dosage forms for oral administration such as capsules and tablets. In particular pediatric and geriatric patients frequently have difficulty in swallowing conventional solid dosage forms. In addition, for many medicaments, the act of swallowing the medicament often requires fluids that increase gastric volume and the likelihood of nausea and vomiting. This occurs more often in hypertension patients. Perhaps the biggest advantage of orally disintegrating dosage forms is that the solid dosage form dissolves or disintegrates quickly in the oral cavity, resulting in a solution or suspension without the need for the administration of fluid. Accordingly, the patient can administer the dosage form as soon as symptoms are felt. Thus, the orally disintegrating dosage form is one of the advantageous methods to deliver the drugs such as comprising angiotensin receptor II antagonists to such patients and provide a better patient compliance with recommended pharmaceutical therapies.

Additionally oral administration of the drugs is difficult in patients having concomitant vomiting, nausea or diarrhoea. The orally disintegrating dosage form is one of the advantageous methods to deliver the drugs to such patients. By administering the orally disintegrating dosage forms, faster absorption of the drug occurs through buccal mucosa and it may reduce the first pass metabolism leading to better efficacy of the drug. This dosage form enhances the clinical effects of some drugs by leading to an increase in bioavailability and a reduction in side effects because of avoidance of first-pass liver metabolism.

WO 99/25321, for instance, discloses immediate or modified release solid oral dosage forms where it is also aimed to provide high drug load tablets. This object is achieved by way of wet granulation resulting in anhydrous eprosartan which is stated to be useful in the preparation of high drug load tablets. In the context of the description, potential disintegrants such as sodium starch glycolate, sodium carmellose and crosslinked polyvinyl pyrrolidone are cited, although a rapid release formulation is not exemplified. The examples further include crospovidone. Among potential diluents were also cited lactose, starch, mannitol, sorbitol, cellulose, inorganic sulfates and phosphates.

In addition to these problems in prior art, some pediatric, geriatric, and psychiatric patient populations exhibit "cheeking" behavior (i.e., holding the oral dosage form in the cheek) to avoid swallowing the medication. Accordingly, orally disintegrating compositions would be desirable to improve patient compliance, particularly among elderly patients, because orally disintegrating compositions are easier to swallow and prevent "cheeking".

It is difficult to develop orally disintegrating compositions because of several different reasons. A satisfied orally disintegrating dosage form needs to meet a number of requirements. Firstly, it has to disintegrate in the mouth spontaneously. The time in which a dosage form must dissolve or disintegrate in the oral cavity is necessarily much shorter than in the stomach. Moreover, a premature release in the mouth could also lead to problems due to the often unpleasant taste of the active ingredient. Besides, these compositions should be very porous and should not be very hard. These porous compositions tend to be very sensitive to humidity. As a consequence, they may have some stability problems.

To fulfill all these requirements the formulation for a specific drug needs to be adapted in particular by a careful selection of the excipients used. However, the excipients selected may lead to formulations which are not bioavailable to the corresponding conventional dosage forms. Thus, they have to be chosen very carefully.

Additionally, precautions have to be taken at the preparation, packaging, handling and storing of the finished dosage forms of orally disintegrating compositions since they tend to be both hygroscopic and friable.

Thus, a need rises and the present invention discloses formulations for orally disintegrating compositions of Compound I or pharmaceutically acceptable salts and/or esters thereof which overcome above described problems and which further provide the advantageous property of allowing the active medicament to disintegrate or dissolve rapidly in the oral cavity which have a pleasant mouth feel and good mechanical strength, enough to be processed in high speed tableting machines and shipped in low cost packages.

### Description of the invention

The main object of the present invention is to provide novel orally disintegrating composition comprising a specific angiotensin II receptor antagonist such as Azilsartan which has a chemical name as 2-ethoxy-1-((2'-(5-oxo-2,5-dihydro-1,2,4-oxadiazol-3yl)-biphenyl-4-yl)methyl)-1H-benzimidazole-7-carboxylic acid (hereinafter reffered as "Compound I") or pharmaceutically acceptable salts and/or esters thereof and one or more pharmaceutically acceptable excipient which overcomes the above described problems in prior art and have additive advantages over them.

According to the invention Compound I is in the form of a medoxomil ester. Preferably, Compound I is in the form of medoxomil ester and potassium salt.

It has unexpectedly been found that the specific combination of the Compound I with the combination of the super disintegrants provides an orally disintegrating composition which avoids the afore-mentioned disadvantages of the orally disintegrating compositions of the prior art.

As used here in, "superdisintegrants" means which swell to many times their original size when placed in water while producing minimal viscosity effects in other words it means a disintegrant which improves disintegrant efficiency resulting in decreased use levels when compared to traditional disintegrants.

According to the invention an orally disintegrating composition of Compound I is provided which is fundamentally comparable with the existing regular conventional solid dosage forms such as tablets or capsules, however with the unexpected benefits found with oral disintegration.

In one embodiment the orally disintegrating composition of Compound I disintegrates in oral cavity in less than 90 seconds, preferably in less than 60 seconds, more preferably in less than 30 seconds.

In another embodiment of this invention, the orally disintegrating composition comprises Compound I or pharmaceutically acceptable salts and/or esters in an amount of 0.1 % to 80.0% by weight of total formulation, preferably in an amount of 1.0 % to 60.0%, the most preferably in an amount of 10.0% to 50.0%. Compound I is preferably in the form of medoxomil salt.

The orally disintegrating compositions of this invention further comprising at least one pharmaceutically acceptable excipient selected from the group comprising super disintegrants, diluents, binders, lubricants, glidants, sweeteners, flavouring agents, preservatives and coloring agents.

Suitable super disintegrants may include but not limited to crospovidone, croscarmellose sodium, low-substituted hydroxypropylcellulose, sodium starch glycollate and the like and mixtures thereof; preferably crospovidone.

It has unexpectedly found that in this orally disintegrating composition having a weight ratio of Compound I to super disintegrants, particularly crospovidone, in the range of between 1:10 and 10:1 (w/w), has a synergistic effect over the disintegration time. Preferably the range is between 1:5 and 5:1 (w/w).

Thus, crospovidone has physical and chemical properties that make it ideal for constituting the appropriate disintegrant for this invention. Because crospovidone particles have a very different appearance from those of the other disintegrants. Crospovidone particles seem to consist of aggregates of smaller particles that are fused together. This aggregation gives crospovidone a spongy, highly porous appearance and it swells very little, yet takes water into its network quite rapidly. This helps crospovidone to dissolve easily and quickly in a little amount of water or saliva and makes its disintegrating rate much faster than other related excipients.

According to this embodiment of the invention, crospovidone is present in an amount of between 0.10 to 30.0 % by weight, preferably in an amount of 3.0 to 15.0 % by weight of the total formulation and the formulation disintegrates in oral cavity in less than 90 seconds, preferably in less than 60 seconds, more preferably in less than 30 seconds.

Suitable diluents may include but not limited to microcrystalline cellulose, mannitol, spray-dried mannitol, lactose, starch, sodium carbonate, sodium bicarbonate, calcium carbonate and the like and mixtures thereof; preferably spray-dried mannitol and/or microcrystalline cellulose.

It is reported that, spray-dried mannitol, has physical chemical properties that make it ideal for constituting the appropriate diluent for this invention. Because it dissolves easily and quickly in a little amount of water or saliva and its disintegrating rate is much faster than powder mannitol and other related saccharine excipients. It is highly compressible and it has optimum fluidity for direct compression processes. It has good dilution capacity due to the size and form of the particle, which makes it possible to accept large amounts of active ingredients that are not easily compressed. It is chemically very stable and non-hygroscopic.

In one embodiment, the amount of spray-dried mannitol is present from 1.0 to 60.% by weight of the orally disintegrating composition, preferably is present from 10.0 to 40.0% by weight of total composition.

In another embodiment, when the amount of microcrystalline cellulose is present from 1.0 to 30.0% by weight of the orally disintegrating composition, preferably it is present from 5.0 to 15.0% by weight, said amount makes it possible to significantly improve mechanical strength of the orally disintegrating tablet such as compressibility and hardness, also reduce friability. Higher quantities have negative impact on the palatability of the formula and lower quantities worsen the mechanical strength of the orally disintegrating tablet.

Surprisingly we have found that when the weight ratio of spray-dried mannitol to microcrystalline cellulose is in the range of between 1:10 and 10:1 (w/w), it has synergistic effect over the mechanical strength such as compressibility of Compound I and achieve a substantial reduction in disintegration time and have a good stability of the orally disintegrating composition. Preferably the range is between 1:5 and 5:1 (w/w).

Suitable binders may include but not limited to polyvinylpyrrolidone, polyethylene glycol, polyvinyl alcohol, cellulose derivatives such as hydroxypropyl methyl cellulose, carboxy methyl cellulose, methyl cellulose, gelatin, polyvinylalcohol, carrageenan, guar gum, xanthan gum and the like and mixtures thereof; preferably the binder is polyvinylpyrrolidone and/or hydroxypropyl methyl cellulose. Binder content is from 1.0 to 20.0%, preferably from 2.0 to 10.0% by weight of total composition.

Suitable lubricants may include but not limited to magnesium strearate, calcium stearate, sodium stearyl fumarate, sodium lauryl sulphate, stearic acid and the like and mixtures thereof; preferably the lubricant is magnesium strearate and sodium lauryl sulfate. Lubricant content is from 0.1 to 10.0% by weight of total composition.

Although the preferred lubricant is magnesium stearate, other less hydrophobic lubricants may be used to counter the hydrophobicity in certain cases such as a combination of magnesium stearate with sodium lauryl sulfate. The preffered ratio is 10:1 to 7:1 (w/w). Suitable glidants may include but not limited to colloidal silicon dioxide, talc, aluminium silicate and the like and mixtures thereof; preferably the glidant is colloidal silicon dioxide. Glidant content is from 0.01 to 5.0% by weight of total composition.

The orally disintegrating compositions of this invention may also include sweeteners and flavouring agents to improve patient compliance.

Suitable sweeteners may include but not limited to aspartame, sucralose, saccharin, sugars such as glucose, lactose, fructose and sugar alcohols such as mannitol, sorbitol, xylitol, erythritol and the like and mixtures thereof; preferably the sweetner is aspartame, sucralose and/or saccharin. Sweetener content is from 0.01 to 5%, preferably from 0.10 to 3.0% by weight of total composition.

Suitable flavoring agents may include but not limited to menthol, peppermint, cinnamon, chocolate, vanillin and fruit essences such as cherry, orange, strawberry, grape etc. and the like and mixtures thereof; preferably the flavouring agent is menthol and/or fruit essences. Flavouring agent content is from 0.01 to 5.0%, preferably from 0.10 to 3.0% by weight of total composition.

In a further embodiment, we have found that menthol, which due to its refreshing effect has a synergic effect with the spray-dried mannitol and a good tastemasking capacity due to its residual effect.

Suitable preservatives may include but not limited to methyl paraben and propyl paraben and their salts (such as sodium, potassium), sodium benzoate, citric acid, benzoic acid, butylated hydroxytoluene and butylated hydroxyanisole and the like and mixtures thereof. Preservative content is from 0,01 to 5.0%, preferably from 0.1 to 2.0% by weight of total composition.

Suiatable colouring agents may include but not limited to ferric oxide, FD&C (Food, Drug & Cosmetic) dyes, poncau and the like and mixtures thereof.

Optionally, a humidity absorbent agent may be added, such as precipitated silica in a proportion from 0.01 to 1.0% in weight of the total weight of the tablet, which may counteract the hydrophobicity of the active ingredient and improve the fluidity of the mixture.

In a further aspect, this present invention provides an orally disintegrating composition of compound I or pharmaceutically acceptable salts and/or esters thereof and one or more pharmaceutically acceptable excipients which is stable throughout the shelflife and which has high bioavailability.

The orally disintegrating compositions of this invention include tablets, sachets, minitablets, multilayer and multicoated tablets, pellets or powders which can be formulated in accordance with methods that are standard in the art. It is preferably in the form of tablets.

In a further aspect, the present invention shows that it is possible to have a significant influence on the disintegration rate of the tablet by modifying the dimensions and shape of the tablet. In general, as the tablet becomes thinner and have higher porosity, the orally disintegrating composition will be weakened faster when it contacts with saliva, because the disintegration process is produced after wetting all the surface of the tablet via capillary action. Also, any shape which maximizes the contact surface with the saliva may produce a significant reduction in disintegration time.

The preferred shape of the orally disintegrating tablet composition of this invention may have a shape of a disk, circle, round, sphere, donut, bar, polygon, ellipse and the like. The preffered shape of the tablet is a flat round shape.

The orally disintegrating compositions of the present invention may be prepared by conventional technology well known to those skilled in the art such as direct compression, dry granulation, wet granulation and the like. The orally disintegrating compositions of the present invention may also be prepared by other technologies such as spray drying, freeze drying, floss formation, molding, Zydis^{®}technology, Flashtab technology, OraSolv^{®} technology, DuraSolv^{®} technology, Wowtab™ (With Out Water quick-dissolve tablet) technology and the like.

Preferably Compound I or pharmaceutically acceptable salts and/or esters thereof, and other excipients are mixed together then the mixture is compressed to form tablets. The manufacturing process is preferably performed by mixing the components, blending the mixture with glidant(s) and lubricant(s) and compressing the blended mixture to form tablets.

The process for preparing the orally disintegrating compositions of the invention comprises the following steps:
(a) mixing Compound I, or pharmaceutically acceptable salts and/or esters thereof with spray-dried mannitol and other excipients, such as super disintegrants, further diluents and binders;
(b) blending the mixture with a glidant and lubricant;
(c) optionally adding other excipients such as sweetners, flavouring agents, preservatives, colouring agents;
(d) compressing the blended mixture to form tablets.

Another embodiment of the present invention is to provide an orally disintegrating pharmaceutical dosage form comprising Compound I with one or more pharmaceutically acceptable excipient for preventing and/or treating hypertension in mammals, particularly in humans.

In another embodiment of this invention Compound I can be administered in combination with diuretics, preferably the diuretics are selected from thiazide derivatives. According to this embodiment, said thiazide derivatives are selected from the group comprising hydrochlorothiazide, methylclothiazide, benzylhydrochlorothiazide, trichlormethiazide, cyclopenthiazide, polythiazide, ethiazide, cyclothiazide, bendroflumethiazide or hydroflumethiazide or mixtures thereof, preferably the thiazide is hydrochlorothiazide.

This invention is further defined by reference to the following example. Although the example is not intended to limit the scope of the present invention, it should be considered in the light of the description detailed above. It will be apparent to those skilled in the art that many modifications, both to materials and methods, may be practiced without departing from the scope of the invention.

To fulfil all these requirements the formulation for a specific drug needs to be adapted in particular by a careful selection of the excipients used. However, the excipients selected may lead to formulations which are not bioavailable to the corresponding conventional dosage forms. Thus, they have to be chosen very carefully.

To minimize the disintegration time and maximise the mechanical strength of the tablets of this invention, this orally disintegrating tablet composition has been designed, made up of the following:

### Example 1

The orally disintegrating tablet composition comprises;
(a) 0.10 to 80.0 % by weight of Compound I or pharmaceutically acceptable salts and/or esters thereof,
(b) 0.10 to 30.0 % by weight of crosscarmellose sodium
(c) 1.0 to 60.0 % by weight of spray-dried mannitol
(d) 1.0 to 30.0 % by weight of microcrystalline cellulose,
(e) 1.0 to 20.0 % by weight of polyvinylpyrollidone,
(f) 0.01 to 5.0 % by weight of colloidal silicon dioxide,
(g) 0.10 to 10.0 % by weight of magnesium stearate and sodium lauryl sulfate.
(h) 0.01 to 5.0 % by weight of saccharin sodium,
(i) 0.01 to 5.0 % by weight of menthol and/or fruit essences.

The formulations of this example are manufactured according to the process described above in the description.

According to standardized methods and equipment for testing friability, hardness and disintegrating time have been provided in European Pharmacopeia. The orally disintegrating tablet formulations of the invention (Ex.1) is tested according to these methods. As it is seen in the Table 1, the hardness of the tablets is quite sufficient to allow easy and convenient removal from the package without breaking the dosage unit. These orally disintegrating tablets are hard enough to be handled and packaged like conventional tablets. They are compressed to a hardness of 20 - 50 Newton and possess a friability of less than 1%. The disintegrating times are acceptable and the taste and mouthfeel of the tablets are good.

Orally disintegrating tablets of the Ex.1 are also tested according to their "Carr compressibility" and "angle of response" as they are also shown in Table 1. Common indices of flowability are the Carr index and the angle of repose. The increase in bulk density of a powder is related to the cohesiveness of a powder. So measurement of the bulk density of a powder is essential to define the flow characteristics. The Carr index gives us the guidance for powder flowability. A lower Carr index of excipients is more desirable for acceptable powder flow. Carr Index Classification and Powder Flowability is shown below;

| **Carr Index (compressibility)** | **(%) Flow** |
|---|---|
| 5 - 12 | Free flowing |
| 12 - 16 | Good |
| 18 - 21 | Fair |
| 23 - 35 | Poor |
| 33 - 38 | Very poor |
| > 40 | Extremely poor |

"Angle of response" is a common method used to measure powder flow with small sample quantity. The angles less than 30° are usually indicative of good flow, while powders with angles greater than 40° are likely to be problematic. The ultimate goal of flow analysis is to identify the powder or powder blend that provides the least weight variation in the finished tablet. The more fluid the powder is, the more efficiently and reproducibly it should fill the die cavities of a tablet press. This more efficient and reproducible die fill should be reflected in increased tablet weights and reduced intertablet weight variation.

**Table 1**

| **Example 1 Test Results** | |
|---|---|
| **Harndness (Newton)** | 20-50 |
| **Friability (%)** | ≤0,45 |
| **Disintegration time (sec)** | 20 |
| **Carr Compressibilty (%)** | 5-12 |
| **Angle of response** | ≤ 30 ° |

## Claims

1. An orally disintegrating composition comprising 2-ethoxy-1-((2'-(5-oxo-2,5-dihydro-1,2,4-oxadiazol-3yl)-biphenyl-4-yl)methyl)-1 H-benzimidazole-7-carboxylic acid (Compound I) or pharmaceutically acceptable salts and/or esters thereof and one or more pharmaceutically acceptable excipients wherein the composition comprises spray-dried mannitol.

2. The orally disintegrating composition according to claim 1, wherein the composition disintegrates in oral cavity in less than 90 seconds, preferably in less than 60 seconds.

3. The orally disintegrating composition according to claims 1 and 2, wherein Compound I or pharmaceutically acceptable salts and/or esters thereof is present in an amount of 0.10 to 80.0% by weight of total composition, preferably it is 1.0 to 60.0% by weight of total composition.

4. The orally disintegrating composition according to claims 1 to 3, wherein Compound I is in the form of a medoxomil ester.

5. The orally disintegrating composition according to claims 1 to 4, wherein Compound I is in the form of a medoxomil ester and potassium salt.

6. The orally disintegrating composition according to claims 1 to 4, wherein the one or more pharmaceutically acceptable excipients are selected from the group comprising super disintegrants, further diluents, binders, lubricants, glidants, sweeteners, flavouring agents, preservatives and coloring agents.

7. The orally disintegrating composition according to claim 6, wherein the super disintegrants are selected from the group comprising crospovidone, croscarmellose sodium, low-substituted hydroxypropylcellulose, sodium starch glycollate and mixtures thereof.

8. The orally disintegrating composition according to claims 6 and 7, wherein the weight ratio of Compound I to super disintegrants is in the range of between 1:10 and 10:1 (w/w).

9. The orally disintegrating composition according to claims 6 and 7, wherein the super disintegrant is preferably crospovidone.

10. The orally disintegrating composition according to claim 9, wherein the amount of crospovidone is present in an amount of 0.10 to 30.0% by weight of total composition, preferably it is 3.0 to 15.0% by weight of total composition.

11. The orally disintegrating composition according to claim 6, wherein the composition further comprises a diluent which is selected from the goup comprising microcrystalline cellulose, mannitol, lactose, starch, sodium carbonate, sodium bicarbonate, calcium carbonate and mixtures thereof; preferably the diluent is microcrystalline cellulose.

12. The orally disintegrating composition according to claim 1, wherein the amount of spray-dried mannitol is in an amount of 1.0 to 60.0% by weight of total composition, preferably it is 10.0 to 40.0% by weight of total composition.

13. The orally disintegrating composition according to claim 11, wherein the amount of microcrystalline cellulose is in an amount of 1.0 to 30.0% by weight of total composition, preferably it is 10.0 to 15.0% by weight of total composition.

14. The orally disintegrating composition according to claims 12 and 13, wherein the weight ratio of spray-dried mannitol to microcrystalline cellulose is in the range of between 1:10 and 10:1 (w/w).

15. The orally disintegrating composition according to claim 6, wherein the binders are selected from the goup comprising polyvinylpyrrolidone, polyethylene glycol, polyvinyl alcohol, cellulose derivatives such as hydroxypropyl methyl cellulose, carboxy methyl cellulose, methyl cellulose, gelatin, polyvinylalcohol, carrageenan, guar gum, xanthan gum and mixtures thereof; preferably polyvinylpyrollidone and/or hydroxypropyl methyl cellulose,

16. The orally disintegrating composition according to claim 6, wherein the lubricants are selected from the goup comprising magnesium strearate, calcium stearate, sodium stearyl fumarate, sodium lauryl sulphate, stearic acid and mixtures thereof; preferably magnesium strearate and sodium lauryl sulfate.

17. The orally disintegrating composition according to claim 16, wherein the weight ratio of magnesium stearate to sodium lauryl sulfate is between 10:1 and 7:1.

18. The orally disintegrating composition according to claim 6, wherein the glidants selected from the goup comprising colloidal silicon dioxide, talc, aluminium silicate and mixtures thereof; preferably colloidal silicon dioxide.

19. The orally disintegrating composition according to claim 6, wherein the sweetners are selected from the goup comprising aspartame, sucralose, saccharin, sugars such as glucose, lactose, fructose and sugar alcohols such as mannitol, sorbitol, xylitol, erythritol and mixtures thereof; preferably aspartame, sucralose and/or saccharin.

20. The orally disintegrating composition according to claim 6, wherein the flavouring agents are selected from the goup comprising menthol, peppermint, cinnamon, chocolate, vanillin and fruit essences such as cherry, orange, strawberry, grape and mixtures thereof; preferably menthol and/or fruit essences.

21. The orally disintegrating composition according to any preceding claim comprising;
(a) 0.10 to 80.0% by weight of Compound I or pharmaceutically acceptable salts and/or esters thereof,
(b) 0.10 to 30.0% by weight of crospovidone,
(c) 1.0 to 60.0% by weight of spray-dried mannitol,
(d) 1.0 to 30.0 % by weight of microcrystalline cellulose,
(e) 1.0 to 20.0% by weight of polyvinylpyrollidone,
(f) 0.01 to 5.0% by weight of colloidal silicon dioxide,
(g) 0.10 to 10.0% by weight of magnesium stearate and sodium lauryl sulfate.
(h) 0.01 to 5.0% by weight of saccharin sodium,
(i) 0.01 to 5.0 % by weight of menthol and/or fruit essences.

22. A process for preparing orally disintegrating compositions according to any preceding claim comprising;
(a) mixing Compound I, or pharmaceutically acceptable salts and/or esters thereof with spray-dried mannitol and other excipients, such as super disintegrants, further diluents and binders;
(b) blending the mixture with a glidant and lubricant;
(c) optionally adding other excipients such as sweeteners, flavouring agents, preservatives, colouring agents;
(d) compressing the blended mixture to form tablets.

23. The orally disintegrating composition according to any preceding claims, for use in preventing and/or treating hypertension in mamals, particulary in humans.

## Patentansprüche

1. Eine oral zerfallende Zusammensetzung umfassend 2-Ethoxy-1-((2'-(5-oxo-2,5-dihydro-1,2,4-oxadiazol-3yl)-biphenyl-4-yl)methyl)-1H-benzimidazol-7-carbonsäure (Verbindung I) oder pharmazeutisch annehmbare Salze davon und/oder Estern davon und ein oder mehrere pharmazeutisch annehmbare Hilfsstoffe, worin die Zusammensetzung sprühgetrocknetes Mannitol umfasst.

2. Die oral zerfallende Zusammensetzung gemäß Anspruch 1, worin die Zusammensetzung in oraler Kavität in weniger als 90 Sekunden zerfällt, vorzugsweise in weniger als 60 Sekunden.

3. Die oral zerfallende Zusammensetzung nach den Ansprüchen 1 und 2, worin die Verbindung I oder pharmazeutisch annehmbare Salze und/oder Ester davon vorhanden ist in einer Menge von 0,10 bis 80,0 Gew.-% der Gesamtzusammensetzung, vorzugsweise ist sie 1,0 bis 60,0 Gew.-% der Gesamtzusammensetzung.

4. Die oral zerfallende Zusammensetzung nach Ansprüchen 1 bis 3, worin die Zusammensetzung I in der Form eines Medoxomilesters ist.

5. Die oral zerfallende Zusammensetzung nach den Ansprüchen 1 bis 4, worin die Verbindung I in der Form eines Medoxomilesters und Kaliumsalzes ist.

6. Die oral zerfallende Zusammensetzung nach den Ansprüchen 1 bis 4, worin der eine oder mehrere pharmazeutisch annehmbare Hilfsstoffe ausgewählt sind aus der Gruppe bestehend aus Super-Sprengmitteln, weiteren Verdünnungsmitteln, Bindemitteln, Schmierstoffen, Gleitmitteln, Süßstoffen, Geschmacksstoffen, Konservierungsmitteln und Farbstoffen.

7. Die oral zerfallende Zusammensetzung nach Anspruch 6, worin die SuperSprengmittel ausgewählt sind aus der Gruppe bestehend aus Crospovidone, Croscarmellosenatrium, niedrig-substituierte Hydroxypropylcellulose, Natriumstärkeglykolat und Mischungen davon.

8. Die oral zerfallende Zusammensetzung nach Ansprüchen 6 und 7, worin das Gewichtsverhältnis der Verbindung I zu den Super-Sprengmitteln in einem Bereich von zwischen 1:10 und 10:1 (w/w) ist.

9. Die oral zerfallende Zusammensetzung gemäß Ansprüchen 6 und 7, worin das SuperSprengmittel vorzugsweise Crospovidone ist.

10. Die oral zerfallende Zusammensetzung nach Anspruch 9, worin die Menge an Crospovidone in einer Menge von 0,10 bis 30,0 Gew.-% der Gesamtzusammensetzung vorliegt, vorzugsweise ist sie 3,0 bis 15,0 Gew.-% der Gesamtzusammensetzung.

11. Die oral zerfallende Zusammensetzung nach Anspruch 6, worin die Zusammensetzung weiterhin umfasst ein Verdünnungsmittel, das ausgewählt ist aus der Gruppe bestehend aus mikrokristalliner Cellulose, Mannitol, Laktose, Stärke, Natriumcarbonat, Natriumbicarbonat, Calciumcarbonat und Mischungen davon, vorzugsweise ist das Verdünnungsmittel mikrokristalline Cellulose.

12. Die oral zerfallende Zusammensetzung nach Anspruch 1, worin die Menge des sprühgetrockneten Mannitols in einer Menge von 1,0 bis 60,0 Gew.-% der Gesamtzusammensetzung ist, vorzugsweise ist sie 10,0 bis 40,0 Gew.-% der Gesamtzusammensetzung.

13. Die oral zerfallende Zusammensetzung nach Anspruch 11, worin die Menge an mikrokristalliner Cellulose eine Menge von 1,0 bis 30,0 Gew.-% der Gesamtzusammensetzung ist, vorzugsweise ist sie 10,0 bis 15,0 Gew.-% der Gesamtzusammensetzung.

14. Die oral zerfallende Zusammensetzung nach Ansprüchen 12 und 13, worin das Gewichtsverhältnis an sprühgetrocknetem Mannitol zu mikrokristalliner Cellulose in einem Bereich von zwischen 1:10 und 10:1 (w/w) ist.

15. Die oral zerfallende Zusammensetzung nach Anspruch 6, worin die Bindemittel ausgewählt sind aus der Gruppe umfassend Polyvinylpyrrolidon, Polyethylenglykol, Polyvinylalkohol, Cellulosederivate wie Hydroxypropylmethylcellulose, Carboxymethylcellulose, Methylcellulose, Gelatine, Polyvinylalkohol, Carrageenan, Guargummi, Xanthangummi und Mischungen davon, vorzugsweise Polyvinylpyrrolidon und/oder Hydroxypropylmethylcellulose.

16. Die oral zerfallende Zusammensetzung nach Anspruch 6, worin die Schmiermittel ausgewählt sind aus der Gruppe umfassend Magnesiumstearat, Calciumstearat, Natriumstearat, Stearylfumarat, Natriumlaurylsulfat, Stearinsäure und Mischungen davon; vorzugsweise Magnesiumstearat und Natriumlaurylsulfat.

17. Die oral zerfallende Zusammensetzung nach Anspruch 16, worin das Gewichtsverhältnis an Magnesiumstearat zu Natriumlaurylsulfat zwischen 10:1 und 7:1 ist.

18. Die oral zerfallende Zusammensetzung nach Anspruch 6, worin die Gleitmittel ausgewählt sind aus der Gruppe umfassend kolloidales Siliziumdioxid, Talk, Aluminiumsilikat und Mischungen davon; vorzugsweise kolloidales Siliziumdioxid.

19. Die oral zerfallende Zusammensetzung nach Anspruch 6, worin die Süßstoffe ausgewählt sind aus der Gruppe umfassend Aspartam, Sucralose, Saccharin, Zucker wie Glukose, Laktose, Fructose und Zuckeralkohol wie Mannitol, Sorbitol, Xylitol, Erythritol und Mischungen davon; vorzugsweise Aspartam, Sucralose und/oder Saccharin.

20. Die oral zerfallende Zusammensetzung nach Anspruch 6, worin die Geschmacksmittel ausgewählt sind aus der Gruppe umfassend Menthol, Pfefferminz, Zimt, Schokolade, Vanillin und Fruchtessenzen wie Kirsche, Orange, Erdbeere, Traube und Mischungen davon, vorzugsweise Menthol und/oder Fruchtessenzen.

21. Die oral zerfallende Zusammensetzung nach irgendeinem der vorhergehenden Ansprüche, umfassend:
(a) 0,10 bis 80,0 Gew.-% an Verbindung I oder pharmazeutisch annehmbarer Salze und/oder Estern davon,
(b) 0,10 bis 30,0 Gew.-% Crospovidone,
(c) 1,0 bis 60,0 Gew.-% sprühgetrocknetes Mannitol,
(d) 1,0 bis 30,0 Gew.-% mikrokristalline Cellulose,
(e) 1,0 bis 20,0 Gew.-% Polyvinylpyrollidon,
(f) 0,01 bis 5,0 Gew.-% kolloidales Siliziumdioxid,
(g) 0,10 bis 10,0 Gew.-% Magnesiumstearat und Natriumlaurylsulfat,
(h) 0,01 bis 5,0 Gew.-% Saccharinnatrium,
(i) 0,01 bis 5,0 Gew.-% Menthol und/oder Fruchtessenzen.

22. Ein Verfahren zur Herstellung oral zerfallender Zusammensetzungen nach irgendeinem der vorherstehenden Ansprüche, umfassend:
(a) Mischen von Verbindung I, oder pharmazeutisch annehmbaren Salzen und/oder Estern davon mit sprühgetrocknetem Mannitol und anderen Hilfsstoffen, sowie Super-Sprengmitteln, weiteren Verdünnungsmitteln und Bindermitteln;
(b) Vermischen der Mischung mit einem Gleitmittel und Schmiermittel;
(c) Hinzugeben weiterer Hilfsstoffe so wie Süßungsmitteln, Geschmacksmitteln, Konservierungsmitteln, Färbungsmitteln;
(d) Komprimieren der vermischten Mischung, um Tabletten zu bilden.

23. Die oral zerfallende Zusammensetzung nach irgendeinem der vorhergehenden Ansprüche, zur Verwendung in der Prävention und/oder Behandlung von Hypertonie in Säugetieren, insbesondere in Menschen.

## Revendications

1. Une composition se désintégrant par voie orale comprenant de l'acide 2-éthoxy-1-[(2'-(5-oxo-2,5-dihydro-1,2,4-oxadiazol-3yl)-biphényl-4-yl)-méthyl]-1H-benzimidazole-7-carboxylique (Composé I) ou des sels et/ou esters acceptables du point de vue pharmaceutique en dérivant et comprenant un ou plusieurs excipients acceptables du point de vue pharmaceutique, ladite composition comprenant du mannitol séché par pulvérisation.

2. La composition se désintégrant par voie orale selon la revendication 1, ladite composition se désintégrant dans la cavité buccale en moins de 90 secondes, de préférence en moins de 60 secondes.

3. La composition se désintégrant par voie orale selon les revendications 1 et 2, dans laquelle le Composé 1, ou des sels et/ou esters acceptables du point de vue pharmaceutique en dérivant, est présent en une proportion de 0,10 à 80,0% en poids de la composition totale, de préférence de 1,0 à 60,0% en poids de la composition totale.

4. La composition se désintégrant par voie orale selon les revendications 1-3, dans laquelle le Composé 1 est sous forme d'un ester de médoxomil.

5. La composition se désintégrant par voie orale selon les revendications 1 à 4, dans laquelle le Composé 1 est sous forme d'un ester de médoxomil et sel de potassium.

6. La composition se désintégrant par voie orale selon les revendications 1 à 4, dans laquelle lesdits uns ou plusieurs excipients acceptables du point de vue pharmaceutique sont choisis dans le groupe comprenant les agents super-désintégrants, et, en outre des diluants, des liants, des lubrifiants, des agents d'écoulement, des agents édulcorants, des agents aromatisants, des agents de conservation et des agents colorants.

7. La composition se désintégrant par voie orale selon la revendication 6, dans laquelle les agents super-désintégrants sont choisis dans le groupe comprenant la crospovidone, la croscarmellose sodique, l'hydroxypropylcellulose faiblement substituée, l'amidon glycolate de sodium et des mélanges de ceux-ci.

8. La composition se désintégrant par voie orale selon la revendication 6 et 7, dans laquelle le rapport pondéral
Composé I / agents super-désintégrants
est dans la gamme comprise entre 1/10 et 10/1 (p/p).

9. La composition se désintégrant par voie orale selon la revendication 6 et 7, dans laquelle l'agent super-désintégrant est de préférence la crospovidone.

10. La composition se désintégrant par voie orale selon la revendication 9, dans laquelle la proportion de crospovidone est présente en une proportion de 0,10 à 30,0% en poids de la composition totale, de préférence en une proportion de 3,0 à 15,0% en poids de la composition totale.

11. La composition se désintégrant par voie orale selon la revendication 6, comprenant en outre un diluant choisi dans le groupe constitué par la cellulose microcristalline, le mannitol, le lactose, l'amidon, le carbonate de sodium, le bicarbonate de sodium, le carbonate de calcium et des mélanges en dérivant; de préférence, le diluant est la cellulose microcristalline.

12. La composition se désintégrant par voie orale selon la revendication 1, dans laquelle la quantité de mannitol séché par pulvérisation est une quantité de 1,0 à 60,0% en poids de la composition totale, de préférence de 10,0 à 40,0% en poids de la composition totale.

13. La composition se désintégrant par voie orale selon la revendication 11, dans laquelle la proportion de cellulose microcristalline est une proportion de 1,0 à 30,0% en poids de la composition totale, de préférence de 10,0 à 15,0% en poids de la composition totale.

14. La composition se désintégrant par voie orale selon les revendications 12 et 13, dans laquelle le rapport, en poids,
mannitol séché par pulvérisation / cellulose microcristalline
est dans la plage comprise entre 1/10 et 10/1 (p/p).

15. La composition se désintégrant par voie orale selon la revendication 6, dans laquelle les liants sont choisis dans le groupe comprenant la polyvinylpyrrolidone, le polyéthylène glycol, l'alcool polyvinylique, les dérivés de cellulose tels que l'hydroxypropylméthylcellulose, la carboxyméthylcellulose, la méthylcellulose, la gélatine, l'alcool polyvinylique, la carraghénine, la gomme de guar, la gomme xanthane et des mélanges de ceux-ci; de préférence la polyvinylpyrrolidone et/ou l'hydroxypropylméthyl cellulose,

16. La composition se désintégrant par voie orale selon la revendication 6, dans laquelle les lubrifiants sont choisis dans le groupe comprenant le stéarate de magnésium, le stéarate de calcium, le stéarylfumarate de sodium, le laurylsulfate de sodium, l'acide stéarique et leurs mélanges; de préférence le stéarate de magnésium et le laurylsulfate de sodium.

17. La composition se désintégrant par voie orale selon la revendication 16, dans laquelle le rapport, en poids,
stéarate de magnésium/laurylsulfate de sodium
est compris entre 10/1 et 7/1.

18. La composition se désintégrant par voie orale selon la revendication 6, dans laquelle les agents d'écoulement sont choisis dans le groupe comprenant le dioxyde de silicium colloïdal, le talc, le silicate d'aluminium et leurs mélanges; de préférence du dioxyde de silicium colloïdal.

19. La composition se désintégrant par voie orale selon la revendication 6, dans laquelle les agents édulcorants sont choisis dans le groupe comprenant l'aspartame, le sucralose, la saccharine, les sucres tels le glucose, le lactose, le fructose et les alcools de sucre tels que le mannitol, le sorbitol, le xylitol, l'érythritol et leurs mélanges; de préférence l'aspartame, le sucralose et/ou la saccharine.

20. La composition se désintégrant par voie orale selon la revendication 6, dans laquelle les agents aromatisants sont choisis dans le groupe comprenant le menthol, la menthe poivrée, la cannelle, le chocolat, la vanilline et les essences de fruits tels que cerise, orange, fraise, raisin et leurs mélanges; de préférence le menthol et/ou les essences de fruits.

21. La composition se désintégrant par voie orale selon l'une quelconque des revendications précédentes, comprenant;
(a) 0,10 à 80,0% en poids de Composé 1 ou des sels et/ou esters acceptables du point de vue pharmaceutique en dérivant,
(b) de 0,10 à 30,0% en poids de crospovidone,
(c) 1,0 à 60,0% en poids de mannitol séché par pulvérisation,
(d) 1,0 à 30,0% en poids de cellulose microcristalline,
(e) 1,0 à 20,0% en poids de polyvinylpyrrolidone,
(f) 0,01 à 5,0% en poids de dioxyde de silicium colloïdal,
(g) de 0,10 à 10,0% en poids de stéarate de magnésium et laurylsulfate de sodium.
(h) 0,01 à 5,0% en poids de saccharine sodium,
(i) 0,01 à 5,0% en poids de menthol et/ou d'essences de fruits.

22. Procédé de préparation de compositions se désintégrant par voie orale selon l'une quelconque des revendications précédentes, comprenant;
(a) le mélange du Composé I, ou des sels et/ou esters acceptables du point de vue pharmaceutique en dérivant, avec du mannitol séché par pulvérisation et d'autres excipients, tels que agents super-désintégrants, et, en outre diluants et liants;
(b) le mélange du mélange précité avec un agent d'écoulement et un lubrifiant;
(c) l'ajout éventuellement d'autres excipients tels que des édulcorants, des agents aromatisants, des conservateurs, des agents colorants;
(d) la compression du mélange obtenu pour former des comprimés.

23. La composition se désintégrant par voie orale selon une des revendications précédentes, pour une utilisation pour la prévention et/ou le traitement de l'hypertension chez les mammifères, particulièrement chez les humains.
